# EUROPEAN PATENT APPLICATION

(11) **EP 1 459 680 A2**
(43) Date of publication of application: **22.09.2004**
(21) Application number: 04251607.0
(22) Date of filing: 19.03.2004
(51) Int. Cl.: A61B 5/024, A61B 8/02

(54) **Method, apparatus and system for deterioration detection of a pulse sensor**

(30) Priority: 20.03.2003 JP 2003078273; 05.03.2004 JP 2004062183
(71) Applicant: SEIKO INSTRUMENTS INC., Chiba-shi, Chiba 261 (JP)
(72) Inventor: Moriya, Koichi Seiko Instruments, Inc., Chiba-shi, Chiba (JP); Ozaki, Toru Seiko Instruments, Inc., Chiba-shi, Chiba (JP); Iijima,Ryuji Seiko Instruments, Inc., Chiba-shi, Chiba (JP)
(74) Representative: Sturt, Clifford Mark

(57) **Abstract**

A deterioration detector, for a pulse detector with which a transmission signal from a transmitting element suffers changes due to the blood stream and the vascular wall to be received in the form of a reflected signal and the resultant reflected signal is processed to obtain a pulse signal, includes a second transmitting element and a second receiving element.
A second transmitting element and a second receiving element are disposed in a deterioration detector so that when a pulse detector having a pulse sensor device constituted by a first transmitting element and a first receiving element is installed in the deterioration detector, the first transmitting element and the second receiving element face each other and the first receiving element and the second transmitting element face each other. A level of a received output signal of the second receiving element when the first transmitting element is driven is compared with a threshold value to thereby allow the progress of deterioration of the first transmitting element to be quantitatively detected. Moreover, data history of the deterioration detector is remotely managed to allow a deterioration detecting system for estimating failure time to be constructed.

## Description

The present invention relates to a structure of a deterioration detector for detecting the progress of deterioration of a pulse sensor device for use in a pulse detector used in a living body information collecting system for detecting living body information of a user in daily life irrespective of the normal/abnormal conditions to manage home health care and emergency notification, a deterioration detecting method, and a deterioration detecting system using the deterioration detector.

Up to now, there is a method in which a transmitting element and a receiving element are used, and a signal as a reflected signal in the form of which a transmission signal from the transmitting element suffers changes due to the blood stream and the vascular wall to be returned back is received/processed in the receiving element to thereby obtain a pulse signal. A pulse detector for obtaining a pulse signal using an ultrasonic wave or light for example as a transmission/reception signal is in the progress of development.

More specifically, with respect to an ultrasonic wave, a pulse detector which is constituted by a transmitting element and a receiving element to obtain a pulse signal on the basis of a quantity of Doppler shift between an inputted ultrasonic wave and a reflected wave is disclosed in the Japanese Unexamined Patent Publication No.2002-085361.

In addition, with respect to light, the Japanese Unexamined Patent Publication No.2003-310580 discloses a biomedical signal detector which is constituted by a transmitting element and a receiving element to obtain a pulse signal on the basis of displacement in intensity between transmitted light and received reflected light. in which a correction coefficient for a measured signal is obtained on the basis of a driving level of the transmitting element when a power supply of the detector is turned ON and a reception level of the receiving element at this time to calculate a pulse signal obtained by multiplying a subsequent measured signal by the correction coefficient,

In the pulse detector described in the Japanese Unexamined Patent Publication No.2002-085361, conventionally, a state of deterioration of the pulse sensor device for detecting a pulse signal is not quantitatively grasped. Thus, there occurs such nonconformity that a signal abruptly becomes unable to be obtained due to a failure of the pulse sensor device during measurement of a pulse. Therefore, a deterioration detector for quantitatively detecting the progress of deterioration is required. Moreover, taking an application to a living body information collecting system regarding the measurement for a long period of time in daily life, construction of a deterioration detecting system for estimating the failure time of a pulse sensor device before occurrence of a failure of the pulse sensor device is also a problem.

In addition, the biomedical signal detector described in the Japanese Unexamined Patent Publication No. 2003-310580 is a detector which is adapted to obtain a correction coefficient inside the detector whenever the power supply is turned ON to calculate measured data using the correction coefficient in the subsequent measurement. Thus, the measured data which a user can look at becomes the data after the correction. That is to say, a user can not verify a state of deterioration of the transmitting element and the receiving element.

In addition, the correction carried out in the biomedical signal detector is only correction using a relative relationship in output value between the transmitting element and the receiving element within the biomedical signal detector. Thus, in a case where the transmitting element and the receiving element are simultaneously, gradually deteriorated, it is impossible to detect an accurate state of deterioration, In other words, this does not lead to detection of true deterioration of the transmitting element or the receiving element.

The present invention has been made in view of the above problems and has an object to provide a deterioration detector, a deterioration detecting method, and a deterioration detecting system for use in a pulse detector (pulse detector using an ultrasonic wave or light, for example) with which a transmission signal from a transmitting element suffers changes due to the blood stream and the vascular wall to be received in the form of a reflected signal and the resultant reflected signal is processed to obtain a pulse signal.

To be specific, a deterioration detector, which is used in a pulse detector including a pulse sensor device having a first transmitting element, and a first receiving element for receiving a reflected signal of a signal transmitted from the first transmitting element, for quantitatively detecting the progress of deterioration of the pulse sensor device, includes a second transmitting element and a second receiving element disposed so as to be paired with the pulse sensor device of the pulse detector, and is characterized in that the second transmitting element and the second receiving element, when the pulse sensor device is installed in the deterioration detector, are disposed so that the first transmitting element included in the pulse sensor device and the second receiving element included in the deterioration detector face each other, and the first receiving element included in the pulse sensor device and the second transmitting element included in the deterioration detector face each other.

In addition, the deterioration detector is characterized by further including: first judgment means for comparing a received output of the second receiving element when the first transmitting element is driven with a threshold value stored in advance to judge the progress of deterioration of the first transmitting element; and second judgment means for comparing a received output of the first receiving element when the second receiving element is driven with a threshold value stored in advance to judge the progress of deterioration of the first receiving element.

In addition, it is characterized in that the threshold value used in the first judgment means or the second judgment means of the deterioration detector is an amplitude of an output voltage waveform of the received signal, or a power spectrum at a specific frequency of the received signal.

In addition, it is characterized in that the deterioration detector is included in a charger of the pulse detector.

Further, a deterioration detecting method of the present invention for use in a pulse detector including a pulse sensor device having a first transmitting element, and a first receiving element for receiving a reflected signal of a signal transmitted from the first transmitting element, for quantitatively detecting the progress of deterioration of the pulse sensor device using a deterioration detector having a second transmitting element and a second receiving element, is characterized by including: disposing the second transmitting element and the second receiving element so that the first transmitting element included in the pulse sensor device and the second receiving element included in the deterioration detector face each other, and the first receiving element included in the pulse sensor device and the second transmitting element included in the deterioration detector face each other; comparing a received output of the second receiving element when the first transmitting element is driven, and/or a received output of the first receiving element when the second transmitting element is driven with a threshold value stored in advance; and judging, on the basis of the comparison results, the progress of deterioration of the first transmitting element, and/or the first receiving element.

In addition, it is characterized in that the threshold value used for the first judgment means or the second judgment means is an amplitude of an output voltage waveform of a signal received in the first or the second receiving element, or a power spectrum at a specific frequency of the signal received in the first receiving element.

Further, a deterioration detecting system of the present invention includes: a living body information terminal for detecting living body information; a deterioration detector for detecting the progress of deterioration of a sensor device used in the living body information terminal for detecting a biomedical signal; a monitoring terminal for receiving detected data from the deterioration detector to record a history of received data; and a central server connected to the monitoring terminal through a public line, and is characterized in the monitoring terminal or the central server estimates a time when the pulse detection becomes unable to be performed on the basis of the history of deterioration data of the sensor device due to the deterioration of the transmitting element or the receiving element.

Here, in the deterioration detector, the time when a pulse becomes unable to be detected due to the deterioration of the transmitting elements or receiving elements may be estimated on the basis of a history regarding deterioration data of the pulse sensor device.

In addition, in the deterioration detecting system, it is characterized in that information of the progress of deterioration of the sensor device, and information of estimated time when living body information becomes unable to be detected are transmitted from the monitoring terminal or the central server to the living body information terminal to be displayed on display means included in the living body information terminal.

Here, information of the progress of deterioration of the sensor device, and information of estimated time when living body information becomes unable to be detected may be transmitted from the deterioration detector to the living body information terminal to be displayed on display means included in the living body information terminal.

Note that with the deterioration detector, the deterioration detecting method, and the deterioration detecting system, it is possible to detect deterioration of the pulse sensor device included in a pulse detector for obtaining a pulse signal using an ultrasonic wave. In this case, a first transmitting piezoelectric element for generating an ultrasonic wave and a first receiving piezoelectric element for receiving the ultrasonic wave will be used as the first transmitting element and the first receiving element of the pulse sensor device of the pulse detector, respectively. Also, a second transmitting piezoelectric element for generating an ultrasonic wave and a second receiving piezoelectric element for receiving the ultrasonic wave will be used as the second transmitting element and the second receiving element included in the deterioration detector, respectively.

In addition, with the deterioration detector, the deterioration detecting method, and the deterioration detecting system, it is possible to detect deterioration of the pulse sensor device included in a pulse detector for obtaining a pulse signal using light. In this case, a first light emitting element for emitting light and a first light receiving element for receiving the light will be used as the first transmitting element and the first receiving element of the pulse sensor device of the pulse detector, respectively. Also, a second light emitting element for emitting light and a second light receiving element for receiving the light will be used as the second transmitting element and the second receiving element included in the deterioration detector, respectively.

In particular, in case of the pulse detector for obtaining a pulse signal using light, there may be adopted such a structure that a first optical element is installed between the first light emitting element and the second light receiving element, and/or a second optical element is installed between the second light emitting element and the first light receiving element, and the light emitted from the respective light emitting elements is introduced, the light emitted from the respective light emitting elements is condensed, an emission direction of the light emitted from the respective light emitting elements is changed, or so forth by the respective optical elements to thereby cause the respective light receiving elements to efficiently receive the light.

With the deterioration detector of the present invention, it is possible to quantitatively detect the progress of deterioration of the pulse device of the pulse detector using an ultrasonic wave or light.

Moreover, the history of the progress of deterioration is managed and processed on the deterioration detector, on a monitoring terminal, or on a central server installed in a remote place, whereby it becomes possible to estimate the failure time of the pulse sensor device before occurrence of the failure. As a result, in the system for collecting living body information on the basis of measurement of a pulse for a long period of time, a situation in which the data measurement becomes impossible due to occurrence of a failure is avoided to allow a more highly reliable living body information collecting system to be realized.

Embodiments of the invention will now be described by way of further example only and with reference to the accompanying drawings, in which:
Fig. 1 is a structural view showing a structure of a deterioration detector showing a first embodiment mode of the present invention;
Fig. 2 is a structural view showing a structure of a pulse sensor device for detecting deterioration with the deterioration detector showing the first embodiment mode of the present invention;
Fig. 3 is a block diagram of the deterioration detector showing the first embodiment mode of the present invention;
Fig. 4 is a flow chart showing a deterioration detecting method implemented in the deterioration detector showing the first embodiment mode of the present invention;
Fig. 5 is a cross sectional view showing a structure of a deterioration detector showing a third embodiment mode of the present invention;
Fig. 6 is a block diagram of another deterioration detector showing the third embodiment mode of the present invention;
Fig. 7 is a flow chart showing a deterioration detecting method implemented in the deterioration detector showing the third embodiment mode of the present invention;
Fig. 8 is a cross sectional view showing a structure of a deterioration detector showing a fourth embodiment mode of the present invention;
Fig. 9 is a cross sectional view showing a structure of a deterioration detector showing a fifth embodiment mode of the present invention;
Fig. 10 is a cross sectional view showing a structure of a deterioration detector showing a sixth embodiment mode of the present invention;
Fig. 11 is a cross sectional view showing a structure of a deterioration detector showing a seventh embodiment mode of the present invention; and
Fig. 12 is a view showing a configuration of a deterioration detecting system of the present invention.

### First Embodiment

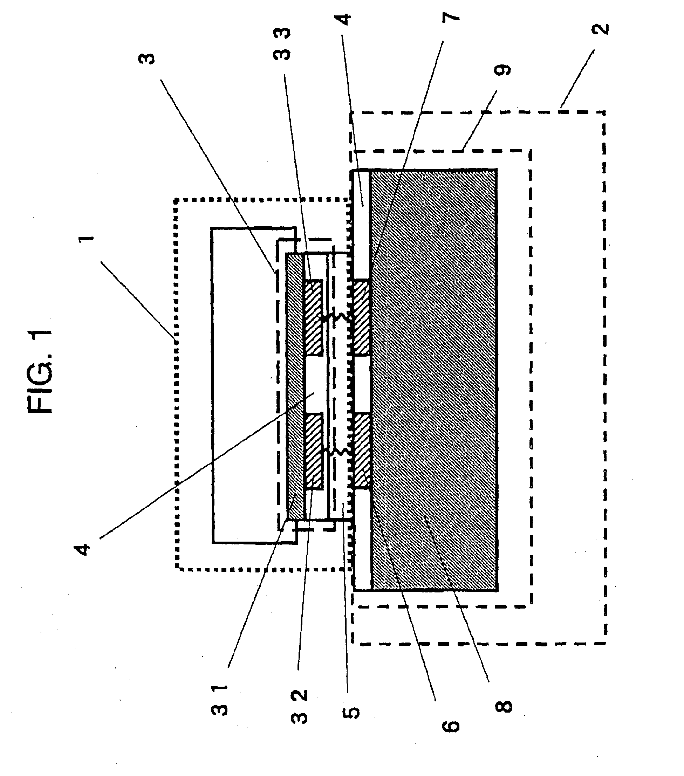

Fig. 1 is a view for explaining a first embodiment mode, i.e., a cross sectional view showing a structure of a deterioration detector 2 of the present invention.

In Fig. 1, a living body information terminal 1 is a wrist type pulse detector for detecting a pulse rate using an ultrasonic wave, and is adapted to detect a pulse with a pulse sensor device 3 as will be described later.

The deterioration detector 2 includes: a deterioration detecting unit 9 having a second transmitting piezoelectric element 7, a second receiving piezoelectric element 6, a support substrate 8 for supporting the second transmitting piezoelectric element 7 and the second receiving piezoelectric element 6, and an acoustic impedance matching layer 4; deterioration detecting unit control means (not shown) for controlling the deterioration detecting unit 9; and display means (not shown) for displaying thereon deterioration detection results. The second transmitting piezoelectric element 7 and the second receiving piezoelectric element 6 are disposed so that when the pulse sensor device is installed in the deterioration detector 2, the second receiving piezoelectric element 6 and a first transmitting piezoelectric element 32 of the pulse sensor device 3 face each other, and the second transmitting piezoelectric element 7 and a first receiving piezoelectric element 33 of the pulse sensor device 3 face each other. Also, the second transmitting piezoelectric element 7 and the second receiving piezoelectric element 6 are structured so as to be fixed by the acoustic impedance matching layer 4. A material of the acoustic impedance matching layer 4 is not limited as long as its acoustic impedance lies between an acoustic impedance of the human body, and an acoustic impedance of the first transmitting piezoelectric element 32 and the first receiving piezoelectric element 33. Thus, as for the material of the acoustic impedance matching layer 4, for example, an epoxy based resin or a silicon based resin may be adopted.

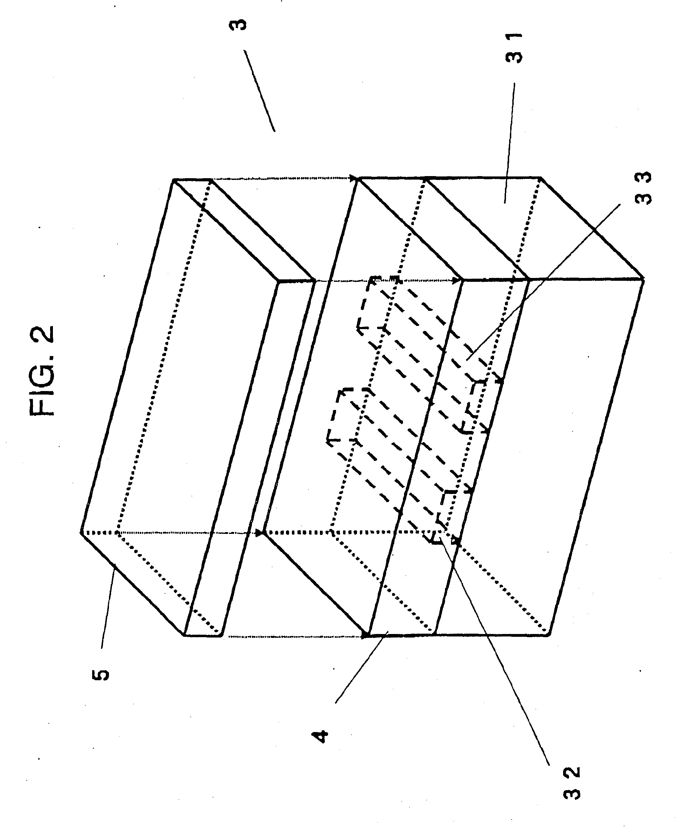

A description will hereinafter be given with respect to the pulse sensor device 3 used in the present invention with reference to Fig. 2.

The pulse sensor device 3 is structured so that the first transmitting piezoelectric element 32 and the first receiving piezoelectric element 33 are disposed side by side on a substrate 31, and moreover are fixed by the acoustic impedance matching layer 4.

In order to detect a pulse, a surface of the pulse sensor device opposite to a substrate adhesion surface of the pulse sensor device 3 is disposed on a portion on a skin of a wrist where the pulse can be detected, and the pulse is measured in a state in which an adherence layer 5 is sandwiched between the pulse sensor device 3 and the skin. The adherence layer 5 is a layer for preventing an ultrasonic wave generated from the first transmitting piezoelectric element 32 and an ultrasonic wave received by the first receiving piezoelectric element 33 from being attenuated. As for a material of the adhesion layer 5, for example, silicon rubber or silicon gel can be adopted.

A reflected wave in the form of which an ultrasonic wave (transmission wave) generated from the first transmitting piezoelectric element 32 is transmitted through the adherence layer 5 to be transmitted through a skin and fat of the human body, and is then reflected from the blood stream in the blood vessels is detected by the first receiving piezoelectric element 33. A pulse signal is obtained on the basis of a quantity of Doppler shift of the reflected wave obtained through the reflection from the blood stream with respect to the transmission wave, and the pulse signal is signal-processed to allow the pulse rate to be detected.

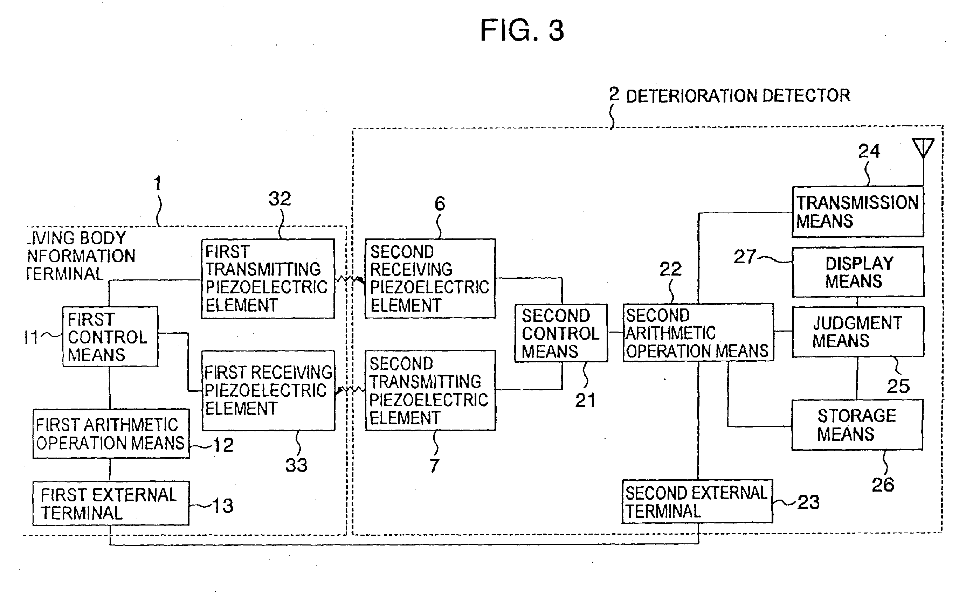

Fig. 3 is a block diagram showing a configuration of the deterioration detector 2 of the present invention. The configuration of the deterioration detector 2 of the present invention will hereinafter be described with reference to Fig. 3.

The deterioration detector 2 includes: a second receiving piezoelectric element 6; a second transmitting piezoelectric element 7; second control means 21 for controlling operations of the second receiving piezoelectric element 6 and the second transmitting piezoelectric element 7; a second external terminal 23 disposed so as to be able to be connected to a first external terminal 13 of the living body information terminal 1; judgment means 25 which will be described later; display means 27 for displaying thereon judgment results obtained from the judgment means 25; storage means 26 constituted by a ROM and a RAM (not shown) which will be described later; transmission means 24 for transmitting data stored in the storage means 26 to the outside; and a second arithmetic operation means 22 for operating the second control means 21, the judgment means 25, the storage means 26, and the transmission means 24, and for managing an operation of the first control means 21 connected to a first arithmetic operation means 12 of the living body information terminal 1 through the second external terminal 23 so as to be adapted to control the first transmitting piezoelectric element 32 and the first receiving piezoelectric element 33.

The judgment means 25 judges the progress of deterioration of the first transmitting piezoelectric element 32 or the first receiving piezoelectric element 33 by utilizing a deterioration detecting method which will be described below.

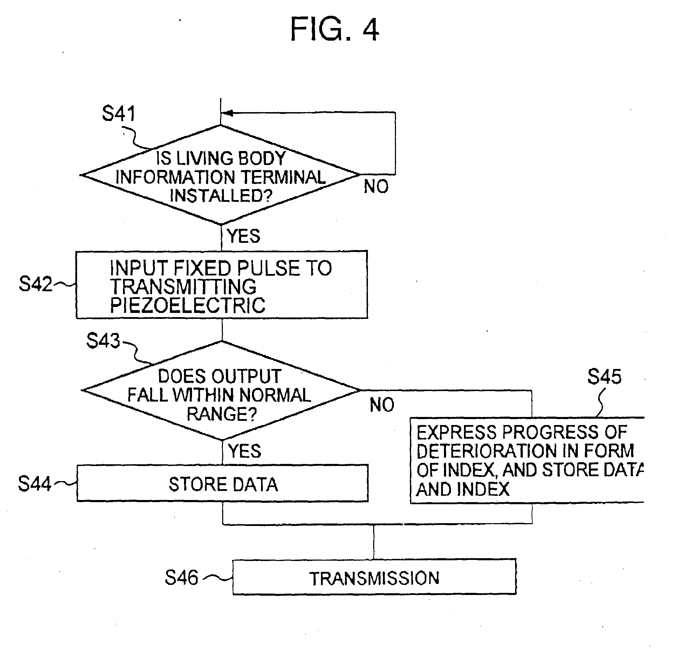

The deterioration detecting method implemented in the judgment means will now be described with reference to Fig. 4.

First of all. in S41, it is verified by living body information terminal installation verifying means (not shown) that the living body information terminal 1 has been installed in the deterioration detector 2. Next, in 542, the control means 21 is controlled so that a pulse having predetermined frequency and power is inputted from the second arithmetic operation means 22 to the first transmitting piezoelectric element 32 included in the living body information terminal 1 or to the second transmitting piezoelectric element 7 included in the deterioration detecting device 2. In S43, the judgment means compares a level of an output signal obtained from the second receiving piezoelectric element 6 or the first receiving piezoelectric element 33 which receives the generated ultrasonic wave, with values in a normal range, as will be described later, stored in the storage means 26 to judge the progress of the first transmitting piezoelectric element and the first receiving piezoelectric element. When it is made clear on the basis of the judgment results that the level of the output signal falls within the normal range, in S44, the measured data, in this case the output value from the first receiving piezoelectric element 33 or from the second receiving piezoelectric element 6, is stored in the storage means 26. On the other hand, when it is made clear on the basis of the judgment results that the level of the output signal lies beyond the normal range, in S45, the progress of deterioration is expressed in the form of an index by referring to a table stored in a ROM in advance which shows a correlation between the measured data and the progress of deterioration to store the resultant index together with the measured data in the storage means 26. Moreover, the contents stored in the storage means 26 are periodically transmitted from the transmission means 24 to the outside in S46.

The normal range is defined as the values between the threshold value, and the level of the output signal obtained from the second receiving piezoelectric element 6 or the first receiving piezoelectric element 33 when the first transmitting piezoelectric element 32 and the second transmitting piezoelectric element 7 are not deteriorated at all.

The threshold value is, for example defined as the value which is equal to 50% of an output signal obtained from the second receiving piezoelectric element 6 or the first receiving piezoelectric element 33 when the first transmitting piezoelectric element 32 and the second transmitting piezoelectric element 7 are not deteriorated at all.

For the judgment with the above-mentioned threshold value, if an amplitude value of an output voltage waveform which is outputted from the second receiving piezoelectric element 6 or the first receiving piezoelectric element 33 when a fixed pulse is inputted to the first transmitting piezoelectric element 32 or the second transmitting piezoelectric element 7, is equal to or larger than 50% of an amplitude value of an output voltage waveform obtained when the first transmitting piezoelectric element 32 and the second transmitting piezoelectric element 7 are not deteriorated at all, then the first transmitting piezoelectric element 32 or the first receiving piezoelectric element 33 is judged to be normal.

In the addition, the threshold value is, for example defined as the value, which is equal to 50% of a peak value of a power spectrum at the frequency characteristics obtained by subjecting to an FFT processing an output voltage waveform which is obtained from the second receiving piezoelectric element 6 or the first receiving piezoelectric element 33 when the first transmitting piezoelectric element 32 and the second transmitting piezoelectric element 7 are not deteriorated at all.

For the judgment with the threshold value, if a peak value of a power spectrum of the frequency characteristics obtained by subjecting to an FFT processing an output voltage waveform which is obtained from the second receiving piezoelectric element 6 or the first receiving piezoelectric element 33 when a fixed pulse is inputted to the first transmitting piezoelectric element 32 or the second transmitting piezoelectric element 7, is equal to or larger than 50% of a peak value of a power spectrum obtained when the first transmitting piezoelectric element 32 or the second transmitting piezoelectric element 7 are not deteriorated at all, then the first transmitting piezoelectric element 32 or the first receiving piezoelectric element 33 is judged to be normal.The storage means 26 is constituted by a ROM and a RAM (not shown). In addition to information of the threshold value, an operation program used to operate the deterioration detecting device 2, an operation program used to operate the living body information terminal 1 when the deterioration detector 2 is connected to the living body information terminal 1, information of a signal processing method for a detected signal or the like is stored in the ROM. On the other hand, the judgment results obtained from the judgment means, and information of an amplitude value of an output voltage waveform obtained from the first receiving piezoelectric element 33 or the second receiving piezoelectric element 6, or the information of the power spectrum at a specific frequency obtained by subjecting the said output voltage waveform to the FFT processing for example, are stored in the RAM (not shown). The transmission means 24 periodically transmits the amplitude value or the power spectrum at the specific frequency which is stored in the storage means 26, or the judgment results of the progress of deterioration to the outside. A transmission destination is a home server installed inside a room for example, and the transmission information is transmitted to a remote place through the home server using a public line. In addition, the deterioration detecting device 2 takes a form of a charger of the living body information terminal 1, whereby the progress of deterioration can be detected periodically, e.g. , once a day. Moreover. the judgment results of the progress of deterioration can also be displayed on display means (not shown) included in the living body information terminal 1.

### Second Embodiment

Instead of, as the threshold value used in the deterioration detector 2, using a table showing a correlation between the measured data obtained from the first receiving piezoelectric element 33 or the second receiving piezoelectric element 7, and the progress of deterioration of the pulse sensor device 3, a table showing a correlation between the measured data and the adherence layer 5 made of silicon gel or the like for example is used to thereby enable the quantitative detection of the progress of deterioration of the adherence layer 5 and the estimation of the exchange time for the adherence layer 5.

### Third Embodiment

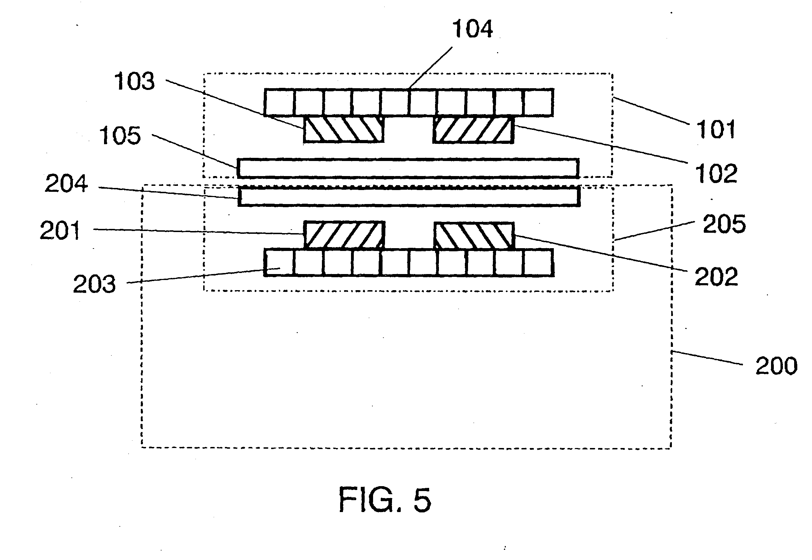

Fig. 5 is a view for explaining a third embodiment mode, i-e. , a cross sectional view showing a structure of a deterioration detecting device 200 of the present invention.

In Fig. 5, a living body information terminal 100 is a wrist type pulse detector for detecting a pulse rate using light, and is adapted to detect a pulse with a pulse sensor device 101 which will be described later.

The deterioration detector 200 includes: a deterioration detecting unit 205 having a second light emitting element 201, a second light receiving element 202, a support substrate 203. for supporting the second light emitting element 201 and the second light receiving element 202, and a cover glass member 204 which is disposed in a light emission direction of the second light emitting element 201 in order to protect the second light emitting element 201 and the second light receiving element 202: deterioration detecting unit control means (not shown) for controlling the deterioration detector; and display means (not shown) for displaying thereon the deterioration detection results. The second light emitting element 201 and the second light receiving element 202 are disposed so that when the pulse sensor device 101 is installed in the deterioration detector 200, the second light receiving element 202 and the first light emitting element 102 of the pulse sensor device 101 face each other, and the second light emitting element 201 and the first light receiving device 103 of the pulse sensor device 101 face each other.

The pulse sensor device 101 used in the present invention will hereinafter be described.

The pulse sensor device 101 has a structure in which the first light emitting element 102 and the first light receiving element 103 are disposed side by side on a substrate 104, the cover glass member 105 for protecting the first light emitting element 102 and the first light receiving element 103 is disposed in a light emission direction of the first light emitting element 102.

In order to detect a pulse, the measurement is carried out in a state in which a surface of the pulse sensor device 101 opposite to a substrate adhesion surface of the pulse sensor device 101 is worn on a wrist.

Light emitted from the first light emitting element 102 is transmitted through the cover glass member 105 to be absorbed and scattered by a skin, fat, muscles, the blood vessels, the blood and the like of the human body. The resultant back-scattered light is then transmitted through the cover glass member 105 to be detected by the first light receiving element 103. The intensity of the detected light fluctuating along with a change in blood volume due to pulsation is signal-processed to allow a pulse rate to be detected.

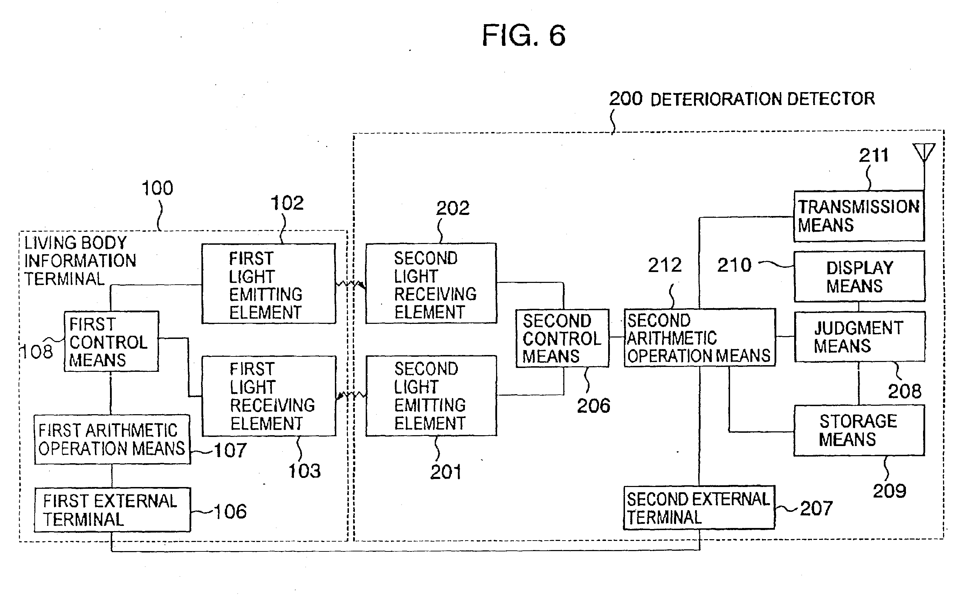

Fig. 6 is a block diagram showing a configuration of the deterioration detector 200 of the present invention. A configuration of the deterioration detector of the present invention will hereinafter be described.

The deterioration detector 200 includes: a second light receiving element 202: a second light emitting element 201; second control means for controlling operations of the second light receiving element 202 and the second light emitting element 201; a second external terminal 207 disposed so as to be able to be connected to a first external terminal 106 of the living body information terminal 100 ; judgment means 208 which will be described later; display means 210 for displaying thereon the judgment results obtained from the judgment means 208; storage means 209 constituted by a ROM and a RAM (not shown) which will be described later; transmission means 211 for transmitting data stored in the storage means 209 to the outside; and second arithmetic operation means 212 which serves to operate the second control means 206, the judgment means 208, the storage means 209, and the transmission means 211, and which is connected to first arithmetic operation means 107 of the living body information terminal 100 through the second external terminal 207 in order to manage an operation of first control means 108 for controlling the first light emitting element 102 and the first light receiving element 103. In this embodiment mode, the external terminal 106 and the external terminal 207 are connected to each other in order to control the living body information terminal 100. However, it is also possible to control the living body information terminal 100 through the light propagation utilizing the first light emitting element 102, the first light receiving element 103, the second light emitting element 201, and the second light receiving element 202.

The judgment means 208 judges the progress of deterioration of the first light emitting element 102 or the first light receiving element 103 by utilizing a deterioration detecting method which will be described later.

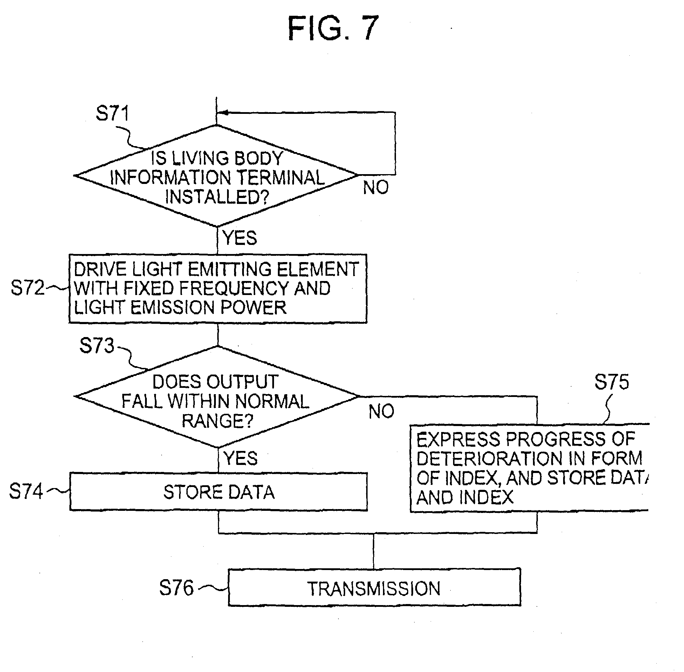

The deterioration detecting method implemented in the judgment means will hereinafter be described with reference to Fig. 7.

First of all, in S71, it is verified by living body information terminal installation verifying means (not shown) that the living body information terminal 100 has been installed in the deterioration detector 200. Next, in S72, the first control means 108 or the second control means 206 is controlled by the second arithmetic operation means 212 so that the first light emitting element 102 included in the living body information terminal 110 or the second light emitting element 201 included in the deterioration detector 200 is driven with predetermined frequency and light emission power. In S73, a level of an output signal obtained from the second light receiving element 202 or the first light receiving element 103 for receiving emitted pulse light is compared with values within a threshold value, as will be described later, stored in the storage means 209 to judge the progress of deterioration of the first light emitting element 102 or the first light receiving element 103. When it is made clear on the basis of the judgment results that the level of the output signal falls within the threshold value, in S74, the measured data, in this case, the output value from the first light receiving element 103 or from the second light receiving element 202, is stored in the storage means 209. On the other hand, when it is made clear on the basis of the judgment results that the level of the output signal lies beyond the threshold value, in S75, the progress of deterioration is expressed in the form of an index by referring to a table showing a correlation between the measured data stored in the ROM in advance and the progress of deterioration. The resultant index is then stored together with the measured data in the storage means 209. Moreover, in S76, the contents stored in the storage means 209 are periodically transmitted to the outside by the transmission means 211.

The normal range is defined as the values between the threshold value, and the level of the output signal obtained from the second light receiving element 202 or the first light receiving element 103 when the first light emitting element 102 and the second light emitting element 201 are not deteriorated at all.

The threshold value is, for example defined as the value which is equal to 50% of an output signal obtained from the second light receiving element 202 or the first light receiving element 103 when the first light emitting element 102 and the second light emitting element 201 are not deteriorated at all.

For the judgment with the threshold value, if an amplitude value of an output voltage waveform which is outputted from the second light receiving element 202 or the first light receiving element 103 when a fixed pulse is inputted to the first light emitting element 102 or the second light emitting element 201. is equal to or larger than 50% of an amplitude value of an output voltage obtained when the first light emitting element 102 and the second light emitting element 201 are not deteriorated at all. then the first light emitting element 102 or the first light receiving element 103 is judged to be normal.

In the addition, the threshold value is, for example defined as the value, which is equal to 50% of a peak value of a power spectrum at the frequency characteristics obtained by subjecting to an FFT processing an output voltage waveform which is obtained from the second light receiving element 202 or the first light receiving element 103 when the first light emitting element 102 and the second light emitting element 201 are not deteriorated at all.

For the judgment with the threshold value, if a peak value of a power spectrum obtained by subjecting to an FFT processing, an output voltage waveform which is outputted from the second light receiving element 202 or the first light receiving element 103 when fixed pulse light is emitted from the first light emitting element 102 or the second light emitting element 201, is equal to or larger than 50% of a peak value of a power spectrum obtained when the first light receiving element 103 and the second light receiving element 202 are not deteriorated at all, then the first light emitting element 102 or the first light receiving element 103 is judged to be normal.

The storage means 209 is constituted by a ROM and a RAM (not shown). In addition to the information of the threshold value, an operation program used to operate the living body information terminal 100 when the deterioration detector 200 and the living body information terminal 100 are connected to each other, information of a method including a signal processing for a detected signal, or the like is stored in the ROM. On the other hand, the judgment results obtained from the judgment means, and information of an amplitude value of an output voltage waveform obtained from the first light receiving element 103 or the second light receiving element 202, or the information of the power spectrum at a specific frequency obtained by subjecting the said output voltage waveform to the FFT processing for example, are stored in the RAM (not shown).

The transmission means 211 periodically transmits information of the amplitude value, or information of a power spectrum at a specific frequency which is stored in the storage means 209, or the judgment results for the progress of deterioration which is stored in the storage means 209 to the outside. A transmission destination, for example, is a home server installed inside a room, and such information is transmitted to a distant place through the home server using a public line. In addition, the deterioration detector 200 takes a form of a charger of the living body information terminal 100, whereby the progress of deterioration can be detected periodically, for example, once a day. Moreover, the judgment results for the progress of deterioration can be displayed on display means (not shown) included in the living body information terminal 100.

### Fourth Embodiment

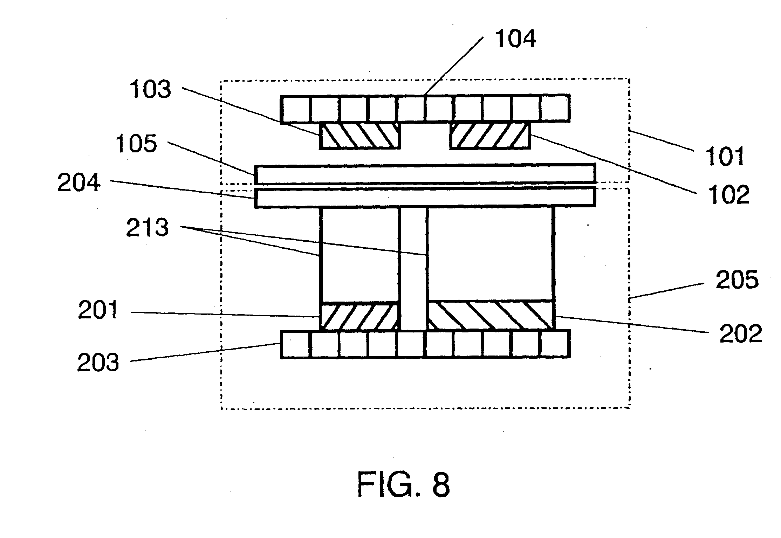

Fig. 8 is a view for explaining a fourth embodiment mode, i.e., a cross sectional view showing a structure of a deterioration detecting unit 205 in the deterioration detector 200 of the present invention.

The deterioration detecting unit 205 includes: a second light emitting element 201; a second light receiving element 202; a support substrate 203 for supporting the second light emitting element 201 and the second light receiving element 202; a cover glass member which is disposed in a light emission direction of the second light emitting element 201 in order to protect the second light emitting element 201 and the second light receiving element 202; and light guiding plates 213 for guiding light emitted from the first light emitting element 102 and light emitted from the second light emitting element 201 to the second light receiving element 202 and the first light receiving element 103, respectively. With such a structure, reflected light of light emitted from the first light emitting element 102 is prevented from reaching the first light receiving element 103, and light emitted from the second light emitting element 201 is prevented from reaching the second light receiving element 202. Moreover, a non-reflection coating is formed on the cover glass member 204, whereby the reflected light of the light emitted from the first light emitting element 102 is prevented from reaching the first light receiving element 103, and the light emitted from the second light emitting element 201 is prevented from reaching the second receiving element 202. As a result, it becomes possible to simultaneously inspect the first light emitting element 102 and the first light receiving element 103.

### Fifth Embodiment

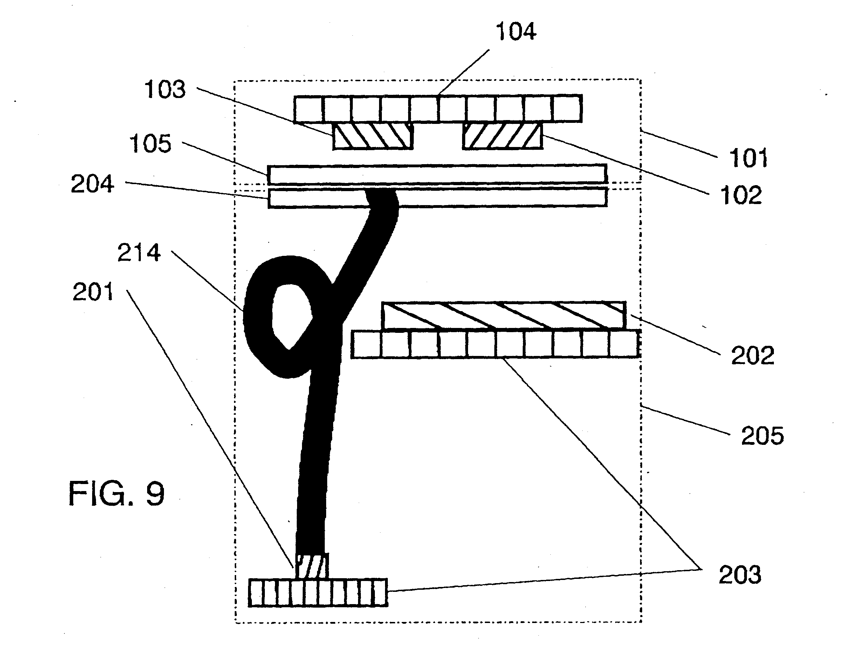

Fig. 9 is a view for explaining a fifth embodiment mode, i.e., a cross sectional view showing a structure of a deterioration detecting unit 205 in the deterioration detector 200 of the present invention.

The deterioration detecting unit 205 includes : a second light emitting element 201; a second light receiving element 202; a support substrates 203 for supporting the second light emitting element 201 and the second light receiving element 202, respectively; a cover glass member which is disposed in a light emission direction of the second light emitting element 201 in order to protect the second light emitting element 201 and the second light receiving element 202; and an optical fiber 214 for guiding light emitted from the second light emitting element 201 to the first light receiving element 103. With such a structure, light emitted from the second light emitting element 201 can reach the first light receiving element 103 without disposing the first light receiving element 103 in a position on a straight line formed together with the first light receiving element 103. Hence, the deterioration can be detected without limiting a size of the light receiving element. A light emission pattern of the light emitting element largely differs depending on light emitting elements. Then, there is adopted a mechanism in which the light emitting element having a large light receiving surface can be used in such a manner, whereby the measurement independent of the light emitting pattern of the light emitting element become possible. While in this embodiment, the optical fiber is provided integrally with the cover glass member, even in case of adoption of a structure in which the optical fiber is provided separately from the cover glass member, the same effects are obtained.

### Sixth Embodiment

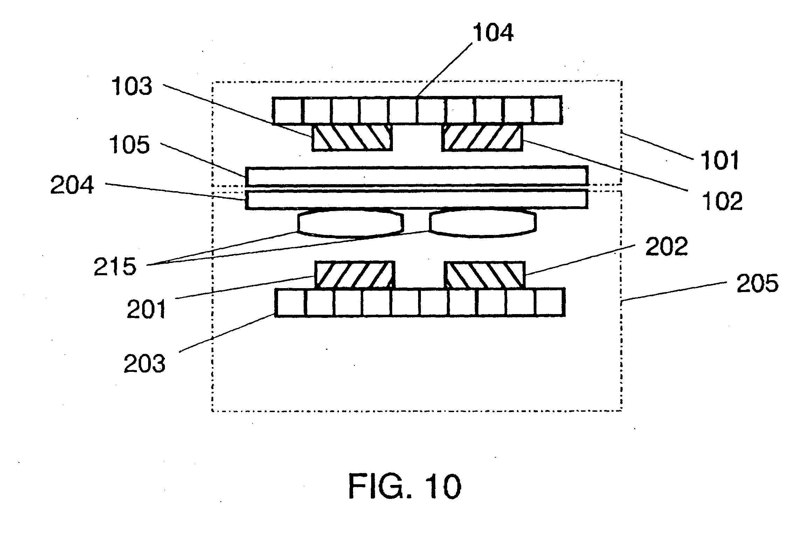

Fig. 10 is a view for explaining a sixth embodiment mode, i. e. , a cross sectional view showing a structure of a deterioration detecting unit 205 in the deterioration detector 200 of the present invention.

The deterioration detecting unit 205 includes: a second light emitting element 201; a second light receiving element 202; a support substrate 203 for supporting the second light emitting element 201 and the second light receiving element 202; a cover glass member 204 which is disposed in a light emission direction of the second light emitting element 201 in order to protect the second light emitting element 201 and the second light receiving element 202; and a right lens 215 for condensing light emitted from the second light emitting element 201 to guide the condensed light to the first light receiving element 103, and a left lens 215 for condensing light emitted from the first light emitting element 102 to guide the condensed light to the second light receiving element 202. The light emitted from one light emitting element and the light emitted from the other light emitting element are condensed using the respective lenses in such a manner, whereby the measurement independent of the light emission pattern of the light emitting element becomes possible. While in this embodiment, the cover glass member is provided integrally with the lenses, even in case of adoption of a structure in which the cover glass member is provided separately from the lenses, the same effects are obtained.

### Seventh Embodiment

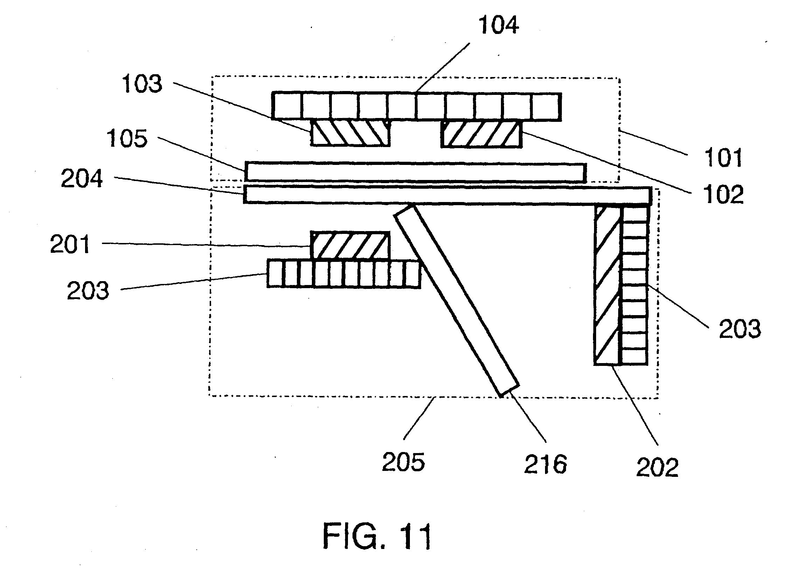

Fig. 11 is a view for explaining a seventh embodiment mode, i.e. , a cross sectional view showing a structure of a deterioration detecting unit 205 in the deterioration detector 200 of the present invention.

The deterioration detecting unit 205 includes: a second light emitting element 201; a second light receiving element 202; support substrates 203 for supporting the second light emitting element 201 and the second light receiving element 202, respectively; a cover glass member 204 which is disposed in a light emission direction of the second light emitting element 201 in order to protect the second light emitting element 201 and the second light receiving element 202: and a reflecting plate 216 for reflecting light emitted from the first light emitting element 102 to guide the reflected light to the second light receiving element 202. In such a manner, the reflected light is guided to the light receiving element using the reflecting plate to allow a position of the light receiving element to be freely set. Hence, it becomes possible to use the light receiving element having a large light receiving surface, and also the measurement independent of the light emission pattern of the light emitting element becomes possible. In this embodiment, the reflecting plate merely reflects the light emitted from the first light emitting element 102. However, a reflecting plate for reflecting the light emitted from the second light emitting element 201 is used, whereby the light emitted from the second light emitting element 201 can reach the first light receiving element 103 without disposing the second light emitting element 201 in a position on a straight line formed together with the first light receiving element 103, and hence the deterioration can be detected without limiting a size of the light receiving element. A light emission pattern of the light emitting element largely differs depending on light emitting elements. Then, there is adopted a mechanism in which the light emitting element having a large light receiving surface can be used in such a manner, whereby the measurement independent of the light emitting pattern of the light emitting element become possible. In addition, while in this embodiment, the reflecting plate is used, even if a prism is used instead of the reflecting plate, the same effects are obtained.

### Eighth Embodiment

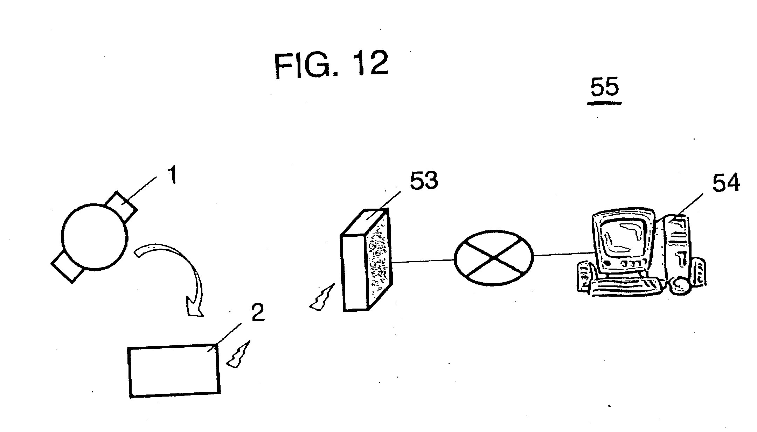

Fig. 12 is a view for explaining an eighth embodiment , i.e. , a view showing a configuration of a deterioration detecting system 55 using the degradation detecting unit 2 of the present invention.

The deterioration detecting system 55 is incorporated in a living body information collecting system for measuring a pulse rate for a long period of time to manage a health state of a wearer. The deterioration detecting system 55 includes: a living body information terminal 1 which is adapted to be carried to detect a pulse; a deterioration detector 2; a monitoring terminal 53 for receiving pulse data transmitted in a wireless manner from the living body information terminal 1, and measured data concerned with the progress of deterioration transmitted in a wireless or wired manner from the deterioration detector 2; and a central sever 54 installed in a remote place for transmitting/receiving data to/from the monitoring terminal 53 through a public line.

Here, the living body information terminal and the deterioration detector are supposed to be the living body information terminal and the deterioration detector described in Embodiment 1, and hence their descriptions are omitted here.

The monitoring terminal 53 periodically (e.g., in a frequency of once a day) receives an amplitude value of an output voltage waveform of the first receiving piezoelectric element 33 or the second receiving piezoelectric element 6, or information of a power spectrum at a specific frequency which is obtained by subjecting said output voltage waveform to the FFT processing, and data which is obtained by expressing the progress of deterioration in the form of an index from the deterioration detector 2 to record these data in the form of history data.

The history data is processed in processing means (not shown) included in the monitoring terminal 53, or is processed in processing means (not shown) included in the central server 54 after being transmitted to the central server 54, so that the failure time of the pulse sensor device included in the living body information terminal 1 is estimated.

Information of the estimated failure time is transmitted three days before a failure estimated day for example from the monitoring device 53 or the central server 54 to the living body information terminal 1 or the deterioration detector 2 to be displayed on display means (not shown) of the living body information terminal 1 or the deterioration detector 2, or to be reported to a wearer by report means (not shown).

Note that the living body information terminal and the deterioration detector may be the living body information terminal 100 and the deterioration detector 200 described in any one of Embodiments 3 to 7.

In addition, the deterioration detector 2 may take a form of a charger of the living body information terminal 1 and may detect the progress of deterioration periodically, e.g., once a day to provide measured data concerned with the progress of deterioration for the monitoring terminal 53.

Also, the processing for the history data may also be executed in the deterioration detector 2 in addition to the monitoring terminal 53 or the central sever 54. In this case, the information of the estimated failure time is displayed on display means (not shown) of the deterioration detector 2 or on display means (not shown) of the living body information terminal 1. The communication between the deterioration detector 2 and the living body information terminal 1 can be made using the first external terminal 13 of the living body information terminal 1 and the second external terminal 23 of the deterioration detector 2.

With the deterioration detecting system as described above, a wearer can estimate the failure time of the pulse sensor device before occurrence of a failure. Moreover, it is possible to avoid a situation in which data becomes unable to be measured due to a failure in long-time measurement of a pulse rate.

## Claims

1. A deterioration detector for a pulse detector comprising a pulse sensor device comprising a first transmitting element and a first receiving element for receiving a reflected signal transmitted from the first transmitting element,
the deterioration detector being used for quantitatively detecting progress of deterioration of the pulse sensor device,
the deterioration detector comprising a second transmitting element and a second receiving element, which are disposed so as to be paired with the pulse sensor device of the pulse detector,
wherein, when the pulse sensor device is installed in the deterioration detector, the second transmitting element and the second receiving element are disposed so that the first transmitting element provided in the pulse sensor device and the second receiving element provided in the deterioration detector face each other, and the first receiving element provided in the pulse sensor device and the second transmitting element provided in the deterioration detector face each other.

2. A deterioration detector according to claim 1, further comprising a first judgment means for comparing a received output of the second receiving element when the first transmitting element is driven with a threshold value stored in advance in the first judgement means, to thereby judge progress of deterioration of the first transmitting element.

3. A deterioration detector according to claim 1, further comprising a second judgment means for comparing a received output of the first receiving element when the second receiving element is driven with a threshold value stored in advance in the second judgment means, to thereby judge the progress of deterioration of the first receiving element.

4. A deterioration detector according to claim 2, wherein the threshold value used in the first judgment means of the deterioration detector is a value obtained by multiplying an amplitude of an output voltage waveform off the received signal at a time of an initial operation by a predetermined coefficient.

5. A deterioration detector according to claim 3, wherein the threshold value used in the second judgment means of the deterioration detector is a value obtained by multiplying an amplitude of an output voltage waveform of the received signal at a time of an initial operation by a predetermined coefficient.

6. A deterioration detector according to claim 2, wherein the threshold value used in the first judgment means of the deterioration detector is a value obtained by multiplying a power spectrum at a specific frequency of the received signal at a time of an initial operation by a predetermined coefficient.

7. A deterioration detector according to claim 3, wherein the threshold value used in the second judgment means of the deterioration detector is a value obtained by multiplying a power spectrum at a specific frequency of the received signal at a time of an initial operation by a predetermined coefficient.

8. A deterioration detector according to claim 1, wherein the deterioration detector is provided in a charger of the pulse detector.

9. A deterioration detection method for a pulse detector comprising a pulse sensor device comprising a first transmitting element and a first receiving element for receiving a reflected signal transmitted from the first transmitting element,
the deterioration detection method being used for quantitatively detecting progress of deterioration of the pulse sensor device using a deterioration detector comprising a second transmitting element and a second receiving element,
the deterioration detection method comprising:
disposing such that the first transmitting element provided in the pulse sensor device and the second receiving element provided in the deterioration detector face each other, and the first receiving element provided in the pulse sensor device and the second transmitting element provided in the deterioration detector face each other;
comparing at least one of a received output of the second receiving element when the first transmitting element is driven and a received output of the first receiving element when the second transmitting element is driven with a threshold value stored in advance in the deterioration detector; and
judging progress of deterioration of at least one of the first transmitting element and the first receiving element.

10. A deterioration detection method according to claim 9, wherein the threshold value stored in advance in the deterioration detector is a value obtained by multiplying at least one of an amplitude of an output voltage waveform of a signal received by the first receiving element and an amplitude of an output voltage waveform of a signal received by the second receiving element at a time of an initial operation by a predetermined coefficient.

11. A deterioration detection method according to claim 9, wherein the threshold value stored in advance in the deterioration detector is a value obtained by multiplying at least one of a power spectrum at a specific frequency of a signal received by the first receiving element and a power spectrum at a specific frequency of a signal received by the second receiving element at a time of an initial operation by a predetermined coefficient.

12. A deterioration detecting system comprising: a living body information terminal for detecting living body information; a deterioration detector for detecting progress of deterioration of a sensor device used in the living body information terminal for detecting a biomedical signal; a monitoring terminal for receiving detected data from the deterioration detector to record a history of received data; and a central server connected to the monitoring terminal through a public line,
wherein one of the monitoring terminal and the central server estimates a future deterioration state of the sensor device based on a history of deterioration data of the sensor device.

13. A deterioration detecting system according to claim 12, wherein the deterioration detector further estimates a future deterioration state of the sensor device based on the history of the deterioration data of the sensor device.

14. A deterioration detecting system according to claim 12, wherein the living body information terminal is a pulse detector comprising a pulse sensor device, and the deterioration detector quantitatively detects the progress of deterioration of the pulse sensor device.

15. A deterioration detecting system according to claim 12, wherein information of the progress of deterioration of the sensor device and information of estimated time when living body information becomes unable to be detected are transmitted from the monitoring terminal or the central server to the living body information terminal to be displayed on display means provided in the living body information terminal.

16. A deterioration detecting system according to claim 13, wherein information of the progress of deterioration of the sensor device and information of estimated time when living body information becomes unable to be detected are transmitted from the deterioration detector to the living body information terminal to be displayed on display means provided in the living body information terminal.

17. A deterioration detecting system according to claim 12, wherein the pulse sensor device has a first transmitting piezoelectric element and a first receiving piezoelectric element for receiving a reflected wave of a transmission wave generated from the first transmitting piezoelectric element, and the deterioration detector has a second transmitting piezoelectric element and a second receiving piezoelectric element.

18. A deterioration detecting system according to claim 12, wherein the pulse sensor device has a first light emitting element and a first light receiving element for receiving reflected light of light emitted from the first light emitting element, and the deterioration detector has a second light emitting element and a second light receiving element.

19. A deterioration detector for a pulse detector comprising a pulse sensor device comprising a first transmitting piezoelectric element for generating an ultrasonic wave and a first receiving piezoelectric element for receiving a reflected wave of the ultrasonic wave transmitted from the first transmitting piezoelectric element,
the deterioration detector being used for quantitatively detecting progress of deterioration of the pulse sensor device,
the deterioration detector comprising:
a second transmitting piezoelectric element and a second receiving piezoelectric element disposed so as to be paired with the pulse sensor device of the pulse detector,
wherein the second transmitting piezoelectric element and the second receiving piezoelectric element are disposed so that the first transmitting piezoelectric element provided in the pulse sensor device and the second receiving piezoelectric element provided in the deterioration detector face each other, and the first receiving piezoelectric element provided in the pulse sensor device and the second transmitting piezoelectric element provided in the deterioration detector face each other.

20. A deterioration detector according to claim 19, further comprising a first judgment means for comparing a received output of the second receiving piezoelectric element when the first transmitting piezoelectric element is driven with a threshold value stored in advance in the judgement means, to thereby judge progress of deterioration of the first transmitting piezoelectric element.

21. A deterioration detector according to claim 19, further comprising a second judgment means for comparing a received output of the first receiving piezoelectric element when the second receiving piezoelectric element is driven with a threshold value stored in advance in the second judgment means, to thereby judge progress of deterioration of the first receiving piezoelectric element.

22. A deterioration detector according to claim 20, wherein the threshold value used for the first judgment means of the deterioration detector is a value obtained by multiplying an amplitude of an output voltage waveform of the received ultrasonic wave at a time of an initial operation by a predetermined coefficient.

23. A deterioration detector according to claim 21, wherein the threshold value used for the second judgment means of the deterioration detector is a value obtained by multiplying an amplitude of an output voltage waveform of the received ultrasonic wave at a time of an initial operation by a predetermined coefficient.

24. A deterioration detector according to claim 20, wherein the threshold value used for the first judgment means of the deterioration detector is a value obtained by multiplying a power spectrum at a specific frequency of the received ultrasonic wave at a time of an initial operation by a predetermined coefficient.

25. A deterioration detector according to claim 21, wherein the threshold value used for the second judgment means of the deterioration detector is a value obtained by multiplying a power spectrum at a specific frequency of the received ultrasonic wave at a time of an initial operation by a predetermined coefficient.

26. A deterioration detector according to any one of claims 18 to 21, wherein the deterioration detector is provided in a charger of the pulse detector.

27. A deterioration detection method, comprising:
comparing a received output of a receiving piezoelectric element when a transmitting piezoelectric element is driven with a threshold value stored in advance in a deterioration detector; and
judging progress of deterioration of the receiving piezoelectric element based on comparison results.

28. A deterioration detection method according to claim 27, wherein the threshold value stored in advance in the deterioration detector is a value obtained by multiplying an amplitude of an output voltage waveform of a received ultrasonic wave at a time of an initial operation by a predetermined coefficient.

29. A deterioration detection method according to claim27, wherein the threshold value stored in advance in the deterioration detector is a value obtained by multiplying a power spectrum at a specific frequency of a received ultrasonic wave at a time of an initial operation by a predetermined coefficient.

30. A deterioration detection method for a pulse detector comprising a pulse sensor device comprising a first transmitting piezoelectric element and a first receiving piezoelectric element for receiving a reflected signal of a signal transmitted from the first transmitting piezoelectric element,
the deterioration detection method being used for quantitatively detecting progress of deterioration of the pulse sensor device using a deterioration detector having a second transmitting piezoelectric element and a second receiving piezoelectric element,
the deterioration detection method comprising:
disposing such that the first transmitting piezoelectric element provided in the pulse sensor device and the second receiving piezoelectric element provided in the deterioration detector face each other, and the first receiving piezoelectric element provided in the pulse sensor device and the second transmitting piezoelectric element provided in the deterioration detector face each other;
comparing at least one of a received output of the second receiving piezoelectric element when the first transmitting piezoelectric element is driven and a received output of the first receiving piezoelectric element when the second transmitting piezoelectric element is driven with a threshold value stored in advance in the deterioration detector; and
judging progress of deterioration of: at leas one of the first transmitting piezoelectric element and the first receiving piezoelectric element.

31. A deterioration detection method according to claim 30, wherein the threshold value stored in advance in the deterioration detector is a value obtained by multiplying at least one of an amplitude of an output voltage waveform of a signal received by the first receiving piezoelectric element and an amplitude of an output voltage waveform of a signal received by the second receiving piezoelectric element at a time of an initial operation by a predetermined coefficient.

32. A deterioration detection method according to claim 30.
wherein the threshold value stored in advance in the deterioration detector is a value obtained by multiplying at least one of a power spectrum at a specific frequency of a signal received by the first receiving piezoelectric element and a power spectrum at a specific frequency of a signal received by the second receiving piezoelectric element at a time of an initial operation by a predetermined coefficient.

33. A deterioration detector for a pulse detector comprising a pulse sensor device comprising a first light emitting element and a first light receiving element for receiving a reflected light transmitted from the first light emitting element,
the deterioration detector being used for quantitatively detecting progress of deterioration of the pulse sensor device,
the deterioration detector comprising:
a second light emitting element and a second light receiving element disposed so as to be paired with the pulse sensor device of the pulse detector,
wherein, when the pulse sensor device is installed in the deterioration detector, the second light emitting element and the second light receiving element are disposed so that the first light emitting element provided in the pulse sensor device and the second light receiving element provided in the deterioration detector face each other, and the first light receiving element provided in the pulse sensor device and the second light emitting element provided in the deterioration detector face each other.

34. A deterioration detector for a pulse detector comprising a pulse sensor device comprising a first light emitting element and a first light receiving element for receiving reflected light emitted from the first light emitting element,
the deterioration detector being used for quantitatively detecting progress of deterioration of the pulse sensor device,
the deterioration detector comprising:
a second light emitting element and a second light receiving element disposed so as to be paired with the pulse sensor device of the pulse detector.
at least one of a first optical element and a second optical element,
the first optical element performs one of guiding the light emitted from the first light emitting element, condensing the emitted light, and changing a light emission direction of the emitted light so that that the light emitted from the first light emitting element is received by the second light receiving element,
the second optical element performs one of guiding the light emitted from the second light emitting element, condensing the emitted light, and changing a light emission direction of the emitted light so that the light emitted from the second light emitting element is received by the first light receiving element.

35. A deterioration detector according to claim 34, wherein the first optical element is one member selected from the group consisting of a light guiding plate, an optical fiber, a reflecting plate, a lens, and a prism.

36. A deterioration detector according to claim 34, wherein the second optical element is one member selected from the group consisting of a light guiding plate, an optical fiber, a reflecting plate, a lens, and a prism.

37. A deterioration detector according to claim 33 or 34, further comprising a first judgment means for comparing a received light output of the second light receiving element when the first light emitting element is driven with a threshold value stored in advance in the first judgement means, to thereby judge progress of deterioration of the first light emitting element.

38. A deterioration detector according to claim 33 or 34, further comprising a second judgment means for comparing a received light output of the first light receiving element when the second light receiving element is driven with a threshold value stored in advance in the second judgement means, to thereby judge progress of deterioration of the second light receiving element.

39. A deterioration detector according to claim 37, wherein the threshold value used for the first judgment means of the deterioration detector is a value obtained by multiplying an amplitude of an output voltage waveform of the received light at a time of an initial operation by a predetermined coefficient.

40. A deterioration detector according to claim 38, wherein the threshold value used for the second judgment means of the deterioration detector is a value obtained by multiplying an amplitude of an output voltage waveform of the received light at a time of an initial operation by a predetermined coefficient.

41. A deterioration detector according to claim 37, wherein the threshold value used for the first judgment means of the deterioration detector is a value obtained by multiplying a power spectrum at a specific frequency of the received light at a time of an initial operation by a predetermined coefficient.

42. A deterioration detector according to claim 38, wherein the threshold value used for the second judgment means of the deterioration detector is a value obtained by multiplying a power spectrum at a specific frequency of the received light at a time of an initial operation by a predetermined coefficient.

43. A deterioration detector according to claim 33, wherein the deterioration detector is provided in a charger of the pulse detector.

44. A deterioration detector according to claim 34, wherein the deterioration detector is provided in a charger of the pulse detector.

45. A deterioration detection method for a pulse detector comprising a pulse sensor device comprising a first light emitting element and a first light receiving element for receiving reflected light emitted from the first light emitting element.
the deterioration detection method being used for quantitatively detecting progress of deterioration of the pulse sensor device using a deterioration detector having a second light emitting element and a second light receiving element,
the deterioration detection method comprising:
disposing such that the first light emitting element provided in the pulse sensor device and the second light receiving element provided in the deterioration detector face each other, and the first light receiving element provided in the pulse sensor device and the second light emitting element provided in the deterioration detector face each other;
comparing at least one of a received light output of the second receiving element when the first light emitting element is driven, and a received light output of the first receiving element when the second light emitting element is driven with a threshold value stored in advance in the deterioration detector; and
judging progress of deterioration of at least one of the first light emitting element and the first receiving element based on comparison results.

46. A deterioration detection method for a pulse detector comprising a pulse sensor device comprising a first light emitting element and a first light receiving element for receiving reflected light emitted from the first light emitting element,
the deterioration detection method being used for quantitatively detecting progress of deterioration of the pulse sensor device using a deterioration detector having a second light emitting element and a second light receiving element,
the deterioration detection method comprising:
providing the deterioration detector with at least one of a first optical element and a second optical element;
receiving light emitted from the first light emitting element by the second light receiving element by one of direct receiving, guiding the emitted light, condensing the emitted light, and by changing a light emission direction of the emitted light using the first optical element;
receiving light emitted from the second light emitting element by the first light receiving element by one of direct receiving, guiding the emitted light, condensing the emitted light, and by changing a light emission direction of the emitted light using the second optical element;
comparing at least one of an output of received light of the second light receiving element when the first light emitting element is driven and an output of received light of the first light receiving element when the second light emitting element is driven with a threshold value stored in advance; and
judging progress of deterioration of at least one of the first light emitting element and the first light receiving element.

47. A deterioration detection method according to claim 46, wherein the first optical element is one member selected from the group consisting of a light guiding plate, an optical fiber, a reflecting plate, a lens, and a prism.

48. A deterioration detection method according to claim 46, wherein the second optical element is one member selected from the group consisting of a light guiding plate, an optical fiber, a reflecting plate, a lens, and a prism.

49. A deterioration detection method according to claims 45, wherein the threshold value stored in advance in the deterioration detector is a value obtained by multiplying at least one of an amplitude of an output voltage waveform of light received by the first light receiving element and an amplitude of an output voltage waveform of light received by the second light receiving element at a time of an initial operation by a predetermined coefficient.

50. A deterioration detection method according to claims 46, wherein the threshold value stored in advance in the deterioration detector is a value obtained by multiplying at least one of an amplitude of an output voltage waveform of light received by the first light receiving element and an amplitude of an output voltage waveform of light received by the second light receiving element at a time of an initial operation by a predetermined coefficient.

51. A deterioration detection method according to claim 45, wherein the threshold value stored in advance in the deterioration detector is a value obtained by multiplying at least one of a power spectrum at a specific frequency of light received by the first receiving element a power spectrum at a specific frequency of light received by the second receiving element at a time of an initial operation by a predetermined coefficient.

52. A deterioration detection method according to claim 46, wherein the threshold value stored in advance in the deterioration detector is a value obtained by multiplying at least one of a power spectrum at a specific frequency of light received by the first receiving element a power spectrum at a specific frequency of light received by the second receiving element at a time of an initial operation by a predetermined coefficient.
